# EUROPEAN PATENT SPECIFICATION

(11) **EP 3 296 745 B1**
(45) Date of publication and mention of the grant of the patent: **30.12.2020**
(21) Application number: 16793048.6
(22) Date of filing: 13.05.2016
(51) Int. Cl.: G01N 33/58, G01N 33/532, G01N 33/569, G01N 27/62, C12Q 1/70, G01N 33/543, G01N 33/576, G01N 33/68, G01N 33/552

(54) **SIMULTANEOUS ANALYSIS METHOD FOR MULTIPLE TARGETS USING MULTIPLE METAL NANO-TAGS**
SIMULTANES ANALYSEVERFAHREN FÜR MEHRFACHE ZIELE UNTER VERWENDUNG MEHRERER METALLNANOTAGS
PROCÉDÉ D'ANALYSE SIMULTANÉE DE PLUSIEURS CIBLES À L'AIDE DE MULTIPLES NANO-ÉTIQUETTES MÉTALLIQUES

(30) Priority: 13.05.2015 KR 20150066820
(43) Date of publication of application: 21.03.2018
(73) Proprietor: SLSBio Co., Ltd., Gyeonggi-do 16229 (KR)
(72) Inventor: MUN, Hae Ran, Seongnam-si Gyeonggi-do 13622 (KR); KIM, Jong Su, Seongnam-si Gyeonggi-do 13508 (KR); KIM, Inae, Yongin-si Gyeonggi-do 16917 (KR)
(74) Representative: J A Kemp LLP
(86) International application number: PCT/KR2016/005116
(87) International publication number: WO 2016/182402

(56) References cited:
- WO-A1-2007/137418
- KR-A- 20140 098 285
- US-A1- 2005 218 319
- US-A1- 2014 308 756
- US-A1- 2015 038 347
- US-B2- 8 481 115
- XING ZHANG ET AL: "Magnetic immunoassay coupled with inductively coupled plasma mass spectrometry for simultaneous quantification of alpha-fetoprotein and carcinoembryonic antigen in human serum", SPECTROCHIMICA ACTA. PART B: ATOMIC SPECTROSCOPY., vol. 106, 1 April 2015 (2015-04-01), pages 20-27, XP055522576, US ISSN: 0584-8547, DOI: 10.1016/j.sab.2015.01.011
- LIU R ET AL: "Sensitive sandwich immunoassay based on single particle mode inductively coupled plasma mass spectrometry detection", TALANTA, ELSEVIER, AMSTERDAM, NL, vol. 83, no. 1, 15 November 2010 (2010-11-15), pages 48-54, XP027449727, ISSN: 0039-9140, DOI: 10.1016/J.TALANTA.2010.08.037 [retrieved on 2010-09-27]
- CHOI H W ET AL: "Cerium oxide-deposited mesoporous silica nanoparticles for the determination of carcinoembryonic antigen in serum using inductively coupled plasma-mass spectrometry", ANALYTICA CHIMICA ACTA, ELSEVIER, AMSTERDAM, NL, vol. 847, 21 August 2014 (2014-08-21), pages 10-15, XP029067144, ISSN: 0003-2670, DOI: 10.1016/J.ACA.2014.08.041
- SHIJIA WU ET AL: "Magnetic nanobead-based immunoassay for the simultaneous detection of aflatoxin Band ochratoxin A using upconversion nanoparticles as multicolor labels", BIOSENSORS AND BIOELECTRONICS, ELSEVIER SCIENCE LTD. UK, AMSTERDAM, NL, vol. 30, no. 1, 16 August 2011 (2011-08-16), pages 35-42, XP028334460, ISSN: 0956-5663, DOI: 10.1016/J.BIOS.2011.08.023 [retrieved on 2011-08-25]
- JUNG AA KO ET AL: "Metal/dye-doped core-shell silica nanoparticles for potential use in bioassay", JOURNAL OF ANALYTICAL ATOMIC SPECTROMETRY, vol. 28, no. 5, 1 January 2013 (2013-01-01), pages 630-636, XP055522658, ISSN: 0267-9477, DOI: 10.1039/c3ja30373j
- GONG, JI - LAI ET AL.: 'Ag/Si02 Core-shell Nanoparticle-based Surface-enhanced Raman Probes for Immunoassay of Cancer Marker using Silica-coated Magnetic Nanoparticles as Separation Tools' BIOSENSORS AND BIOELECTRONICS vol. 22, no. 7, 2007, pages 1501 - 1507, XP022022985
- WU, SHIJIA ET AL.: 'Magnetic Nanobead-based Immunoassay for the Simultaneous Detection of Aflatoxin B1 and Ochratoxin A using Upconversion Nanoparticles as Multicolor Labels' BIOSENSORS AND BIOELECTRONIS vol. 30, no. 1, 2011, pages 35 - 42, XP028334460

## Description

### BACKGROUND OF THE INVENTION

### Field of the invention

The present invention relates to a method for simultaneous analysis of a target using a plurality of metal nano-tags and, more particularly, to a method for simultaneous analysis of a target using a plurality of metal nano-tags, in which the method allows the convergence of a nano-particle technology based on an antigen-antibody reaction, which is a conventional biological immune response, and simultaneously diagnoses a plurality of target materials using a plurality of antigen-antibody reactions and a plurality of metal nano-tags, thereby enhancing diagnostic effect.

### Related Art

Major pathogenic viruses, for example, hepatitis B virus (HBV), hepatitis C virus (HCV), and human immunodeficiency virus (HIV), which are spread through blood or body fluids, are very important prognostic factors in blood management for transfusion.

Currently, the infection diagnosis of these pathogens is mainly performed by an enzyme-linked immunosorbent assay (ELISA), which can detect the presence of HBV surface antigen (HBsAg) or antibodies against HCV and HIV in the blood and thereby determine the presence of virus infection. However, ELISA has a problem in that the accuracy of the assay is low during the latent period until the antibody is formed after virus infection, while it is in a state of infection of viruses with different immune activities, or immune inactivity of the infected person.

In order to overcome the limitations of ELISA, a method that has been developed over the past several years is a nucleic acid test (NAT) which directly detects a genetic material of a virus consisting of DNA or RNA. The nucleic acid test is a diagnostic method for analyzing the presence/absence of a virus with enhanced sensitivity compared to enzyme immunoassay, using an oligo primer with nucleotide sequence specificity for viral nucleic acid. The nucleic acid test was expected to be appropriately utilized for screening pathogenic viruses that are transmitted through the blood in the fields of blood-associated business such as blood transfusion or biopharmaceutical business. However, it is difficult to utilize the nucleic acid test as a routine test method to handle a large amount of specimens due to the problem of cost incurring in the course of introducing and utilizing the test method.

In addition, there is a method called "multiplex NAT" to be used as a method for simultaneously detecting several kinds of viruses. The multiplex NAT has the effect of reducing the inspection time and effort to some extent if introduced. However, the method has difficulties in that the sensitivity may be degraded unless the optimization of the reaction conditions is warranted and that there is a risk of false positive or false negative, and also there is still difficulty in terms of inspection costs to utilize the method as a routine test method to handle a large amount of specimens. Zhang et al (2015. Spectrochimica Acta, 106, 20-27) discloses a method for the simultaneous analysis of target analytes (namely CEA and AFP) by inductively coupled mass spectrometry. The authors use antibodies bound to Au- and Ag-containing silica nanoparticles.

Despite the advantages and disadvantages of various methods for the diagnosis of viruses as described above, the technology primarily used in clinical diagnostics at present to detect viruses is ELISA based on antigen-antibody reaction. Although ELISA method is commonly used because the operation of the measuring machine is simple and the sample can be processed rapidly, the assay has many problems in that the types of usable chromogens or phosphors are limited, there is a difficulty in tagging, reactivity of enzymes related to color development, and in the case of fluorescence, there are various constraints for measurements due to photo bleaching, quenching, etc. In particular, it is even more so in the field of applications where quantitative measurements are required.

Accordingly, there is a need for the development of a novel technology that enables accurate measurement and quantification as well as quantification of target materials such as various kinds of proteins in various matrices.

For this purpose, there is known a method which can detect even a trace amount of virus by using metal nano-tags instead of phosphors or other chromogenic compounds in the conventional ELISA method and measuring the mass of the metal.

However, conventionally, the method using a metal nano-tag has a problem in that it is difficult to simultaneously detect a plurality of targets because it includes only one kind of metal.

### SUMMARY OF THE INVENTION

In order to solve the problems in the conventional technologies, an object of the present invention is to provide a novel method of analysis which enables simultaneous analysis of a plurality of targets using a plurality of antibodies.

The present invention, in order to solve the above objects, provides a method for simultaneous analysis of a plurality of targets using a plurality of metal nano-tags, which includes:
(i) preparing an analysis platform to which a first antibody, that specifically binds to a target, is bound;
(ii) reacting the analysis platform including the first antibody with a sample containing a plurality of targets and thereby forming an analysis platform to which target materials are bound;
(iii) reacting a second antibody, that specifically binds to a target, with the analysis platform in which the first antibody and targets are bound; and
(iv) performing a quantitative analysis of the material to which the second antibody is bound,
wherein: the analysis platform to which the first antibody is bound comprises a plurality of types of antibodies; the analysis platform to which the first antibody is bound is a silica nanoparticle, comprising a metal-comprising core and silica that coats the surface of the core; the target is at least two selected from the group consisting of hepatitis B virus (HBV), hepatitis C virus (HCV), and human immunodeficiency virus (HIV); and the analysis platform to which the first antibody is bound comprises at least two types silica nanoparticles containing different types of metals, wherein the silica nanoparticle comprises Au when the target to be analyzed is HBV, the silica nanoparticle comprises Gd when the target to be analyzed is HIV, and the silica nanoparticle comprises Eu when the target to be analyzed is HCV.

In the method for simultaneous analysis of targets using a plurality of metal nano-tags according to the present invention, the target molecules to be analyzed are viruses.

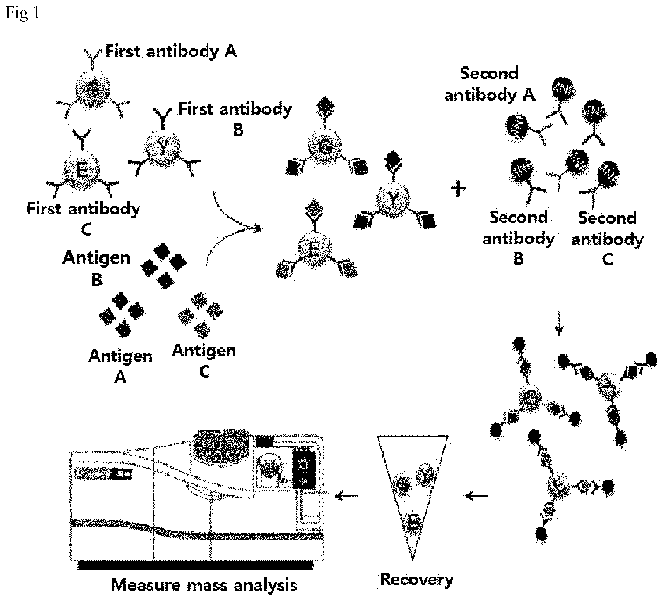

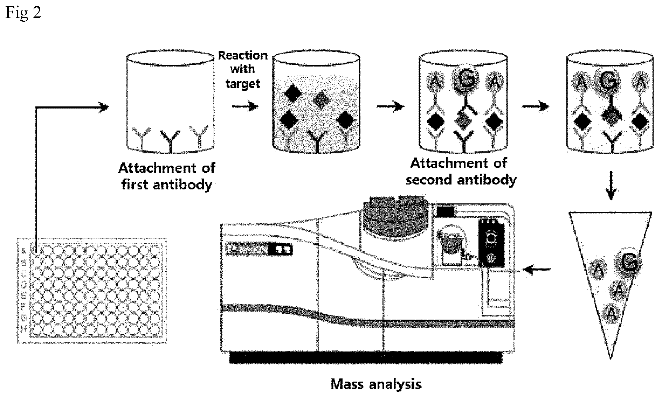

In the method for simultaneous analysis of targets using a plurality of metal nano-tags by the present invention, the analysis platform to which the first antibody is bound is a silica nanoparticle which contains a metal-containing core and silica that coats the surface of the core, or a plate to which a plurality of types of first antibodies are bound. In the method for simultaneous analysis of targets using a plurality of metal nano-tags by the present invention, the case of silica nanoparticle where the analysis platform to which the first antibody is bound is silica nanoparticle which contains a metal-containing core and silica that coats the surface of the core is illustrated in FIG. 1. A case of silica nanoparticle where the analysis platform to which the first antibody is bound is a plate to which a plurality of types of first antibodies are bound is illustrated in FIG. 2.

In the method for simultaneous analysis of targets using a plurality of metal nano-tags by the present invention, each of the silica nanoparticles may be characterized by containing a single kind of metal, and the analysis platform to which the antibody is bound is characterized by containing at least two types of silica nanoparticles with different types of metals. The method for simultaneous analysis of targets using a plurality of metal nano-tags by the present invention is characterized in that the method can diversify the kinds of targets according to the type of the first antibody via analysis by attaching a different type of the first antibody to a plurality of the silica nanoparticles with different types of metals, and the method also enables a simultaneous analysis of two different kinds of metals when quantitative analysis is performed later using an inductively coupled plasma mass spectrometry (ICP-MS) by varying the kinds of the metals contained in the silica nanoparticles.

In the method for simultaneous analysis of targets using a plurality of metal nano-tags by the present invention, the analysis platform to which the first antibody is bound is characterized by comprising at least two types of silica nanoparticles containing different types of metals, wherein the silica nanoparticle comprises Au when the target to be analyzed is HBV, the silica nanoparticle comprises Gd when the target to be analyzed is HIV, and the silica nanoparticle comprises Eu when the target to be analyzed is HCV.

In the method for simultaneous analysis of targets using a plurality of metal nano-tags by the present invention, for the analysis platform to which the first antibody is bound, an appropriate metal is selected depending on the target to be analyzed. Specifically, silica nanoparticle containing Au is desirable when the target to be analyzed is HBV in the blood; silica nanoparticle containing Gd is desirable when the target to be analyzed is HIV in the blood; and silica nanoparticle containing Eu is desirable when the target to be analyzed is HCV in the blood; and it is possible that at least two of these silica nanoparticles are used simultaneously.

In the method for simultaneous analysis of targets using a plurality of metal nano-tags by the present invention, the first antibody may be characterized to be a monoclonal antibody.

In the method for simultaneous analysis of targets using a plurality of metal nano-tags by the present invention, it is possible that the first antibody and the second antibody to be used are the same.

In the method for simultaneous analysis of targets using a plurality of metal nano-tags by the present invention, it is possible that the first antibody is a monoclonal antibody and the second antibody is a polyclonal antibody.

In the method for simultaneous analysis of targets using a plurality of metal nano-tags by the present invention, step (iv) of performing a quantitative analysis of the material to which the second antibody is bound may include:
(iv-1) capturing the target material to which the second antibody is bound by applying an external magnetic force; and
(iv-2) analyzing the captured target material to which the second antibody is bound using a spectrophotometer. That is, in the case of using silica nanoparticles as the analysis platform to which the first antibody is bound, the target to which the second antibody is bound may be separated by the magnetism of the magnetic nanoparticles which are connected to the second antibody, and the tagged metal of the separated target to which even the second antibody is bound is subjected to quantitative analysis for the analysis of the target.

In the method for simultaneous analysis of targets using a plurality of metal nano-tags by the present invention, step (iv-2) of analyzing the captured target material to which the second antibody is bound using a spectrophotometer may be characterized by performing the analysis using an inductively coupled plasma mass spectrometry (ICP-MS) or graphite furnace atomic absorption spectrophotometer.

### BRIEF DESCRIPTION OF THE DRAWINGS

FIGS. 1 and 2 show schematic diagrams illustrating a method for simultaneous analysis of targets using a plurality of metal nano-tags, with FIG 1 being by the present invention.
FIGS. 3 and 4 show the analysis results by ICP-MS in the blood according to an exemplary embodiment of the present invention.

### BEST MODE FOR CARRYING OUT THE INVENTION

The method for simultaneous analysis of a plurality of targets using a plurality of metal nano-tags according to the present invention includes:
(i) preparing an analysis platform to which a first antibody, that specifically binds to a target, is bound;
(ii) reacting the analysis platform including the first antibody with a sample containing a plurality of targets and thereby forming an analysis platform to which target materials are bound;
(iii) reacting a second antibody, that specifically binds to a target, with the analysis platform in which the first antibody and targets are bound; and
(iv) performing a quantitative analysis of the material to which the second antibody is bound,

wherein: the analysis platform to which the first antibody is bound comprises a plurality of types of antibodies; the analysis platform to which the first antibody is bound is a silica nanoparticle, comprising a metal-comprising core and silica that coats the surface of the core; the target is at least two selected from the group consisting of hepatitis B virus (HBV), hepatitis C virus (HCV), and human immunodeficiency virus (HIV); and the analysis platform to which the first antibody is bound comprises at least two types of silica nanoparticles containing different types of metals, wherein the silica nanoparticle comprises Au when the target to be analyzed is HBV, the silica nanoparticle comprises Gd when the target to be analyzed is HIV, and the silica nanoparticle comprises Eu when the target to be analyzed is HCV.

In the method for simultaneous analysis of a plurality of targets using a plurality of metal nano-tags according to the present invention, the analysis platform to which the first antibody is bound may be a silica nanoparticle which contains a magnetic metal-containing core and a silica that coats the surface of the core. The silica nanoparticle may contain a single type of metal; and the analysis platform to which the antibody is bound contains at least two types of silica nanoparticles containing different types of metals. The analysis platform to which the first antibody is bound comprises at least two types of silica nanoparticles containing different types of metals, wherein the silica nanoparticle comprises Au when the target to be analyzed is HBV, the silica nanoparticle comprises Gd when the target to be analyzed is HIV, and the silica nanoparticle comprises Eu when the target to be analyzed is HCV. The second antibody may be bound to a silica nanoparticle, which contains a magnetic metal-containing core and a silica that coats the surface of the core.

In the method for simultaneous analysis of a plurality of targets using a plurality of metal nano-tags according to the present invention, the second antibody may be bound to a silica nanoparticle which contains a magnetic metal-containing core and a silica that coats the surface of the core.

In the method for simultaneous analysis of a plurality of targets using a plurality of metal nano-tags according to the present invention, the core of the silica nanoparticle bound to the second antibody may contain at least two metals selected from the group consisting of Au, Ag, Pt, Pd, Ir, Rh, Ru, Al, Cu, Te, Bi, Pb, Fe, Ce, Mo, Nb, W, Sb, Sn, V, Mn, Ni, Co, Zn, La, Ce, Pr, Nd, Pm, Sm, Eu, Gd, Tb, Dy, Ho, Er, Tm, Yb, Lu, Sc, Y, and Ti.

In the method for simultaneous analysis of a plurality of targets using a plurality of metal nano-tags according to the present invention, the core of the silica nanoparticle bound to the second antibody may contain Au.

In the method for simultaneous analysis of a plurality of targets using a plurality of metal nano-tags according to the present invention, the first antibody and the second antibody may be the same.

In the method for simultaneous analysis of a plurality of targets using a plurality of metal nano-tags according to the present invention, the first antibody may be a monoclonal antibody and the second antibody may be a polyclonal antibody.

In the method for simultaneous analysis of a plurality of targets using a plurality of metal nano-tags according to the present invention,

step (iv) of performing a quantitative analysis of the material to which the second antibody is bound may include:
(iv-1) capturing the target material to which the second antibody is bound by applying an external magnetic force; and
(iv-2) analyzing the captured target material to which the second antibody is bound using a spectrophotometer.

In the method for simultaneous analysis of a plurality of targets using a plurality of metal nano-tags according to the present invention,

step (iv-2) of analyzing the captured target material to which the second antibody is bound using a spectrophotometer may be to analyze using an inductively coupled plasma mass spectrometry (ICP-MS) or graphite furnace atomic absorption spectrophotometer.

### DESCRIPTION OF EXEMPLARY EMBODIMENTS

Hereinafter, the present invention will be described in more detail with reference to examples.

### < Reference Example 1> Case using plate as analysis platform containing first antibody

After attaching a Human anti-p24 monoclonal antibody as a first antibody to a plate, HBsAg was attached as a second antibody to the plate, and silica nanoparticles containing an Au particle were prepared using the Gold Nanoparticle Conjugation kit

A blood sample was allowed to flow through the plate to induce a reaction between the first antibody and a target in the blood sample and unreacted impurities were removed by washing. The HBsAg was attached the resultant and allowed to react with a second antibody containing an Au particle.

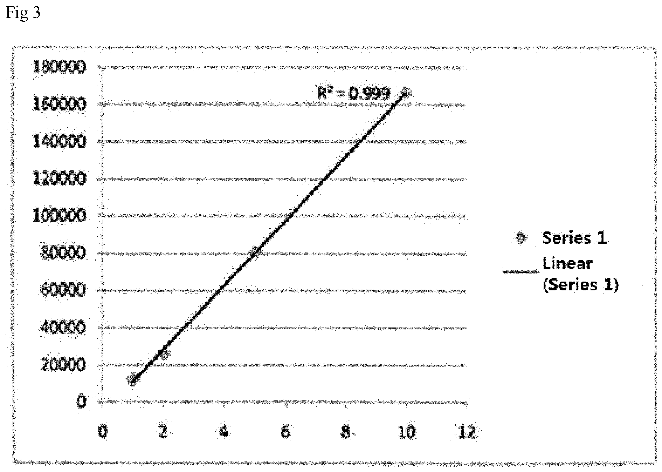

Then, the conjugate bound to the second antibody was separated and recovered by a reaction with nitric acid and the weight of the conjugate was measured using an ICP-MS. The results are shown in FIG. 3.

### <Example 2> Case using silica nanoparticle as analysis platform containing first antibody

Gadolinium-doped silica nanoparticles, yttrium-doped silica nanoparticles, and europium-doped silica nanoparticles were synthesized as an analysis platform containing the first antibody, respectively.

Human anti-p24 monoclonal antibody was attached to each of the synthesized silica nanoparticles as a first antibody and mixed, and thereby an analysis platform containing silica nanoparticles was prepared.

Iron nanoparticles were prepared as magnetic nanoparticles and by attaching human anti-p24 monoclonal antibody thereto as a second antibody.

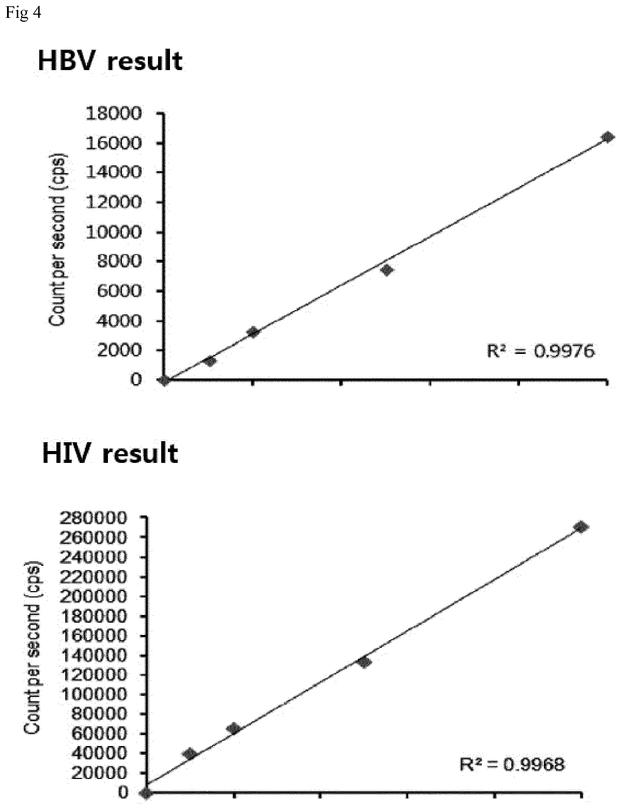

Silica nanoparticles, in which Gadolinium-doped silica nanoparticles, yttrium-doped silica nanoparticles, and europium-doped silica nanoparticles were mixed, were reacted with a sample containing target materials. After removing the unreacted materials, the conjugate bound to the second antibody was separated and recovered by a reaction with nitric acid, and the weight of the resultant was measured by ICP-MS. The results are shown in FIG. 4.

It was confirmed that a plurality of targets can be quantitatively analyzed when a method for simultaneous analysis of a plurality of targets using a plurality of metal nano-tags according to the present invention.

### ADVANTAGEOUS EFFECTS OF INVENTION

The analysis method for a target material using metal nano-tags according to the present invention fuses a nanotechnology to a conventional biological immune response, and the method thereby makes it possible to accurately detect even a trace amount of virus without the burden of inspection cost, in the business of verification/diagnosis of blood preparations, viruses, and other biomedicines as well as in the blood management business which deals with a large amount of blood samples.

## Claims

1. A method for simultaneous analysis of a plurality of targets using a plurality of metal nano-tags, comprising:
(i) preparing an analysis platform to which a first antibody, that specifically binds to a target, is bound;
(ii) reacting the analysis platform comprising the first antibody with a sample comprising a plurality of targets and thereby forming an analysis platform to which target materials are bound;
(iii) reacting a second antibody, that specifically binds to a target, with the analysis platform in which the first antibody and targets are bound; and
(iv) performing a quantitative analysis of the material to which the second antibody is bound,
wherein:
the analysis platform to which the first antibody is bound comprises a plurality of types of antibodies;
the analysis platform to which the first antibody is bound is a silica nanoparticle, comprising a metal-comprising core and silica that coats the surface of the core;
the target is at least two selected from the group consisting of hepatitis B virus (HBV), hepatitis C virus (HCV), and human immunodeficiency virus (HIV); and
the analysis platform to which the first antibody is bound comprises at least two types of silica nanoparticles containing different types of metals, wherein the silica nanoparticle comprises Au when the target to be analyzed is HBV, the silica nanoparticle comprises Gd when the target to be analyzed is HIV, and the silica nanoparticle comprises Eu when the target to be analyzed is HCV.

2. The method of claim 1, wherein the second antibody is bound to a silica nanoparticle, which comprises a magnetic metal-comprising core and silica that coats the surface of the core.

3. The method of claim 1 or 2, wherein the first antibody and the second antibody are the same.

4. The method of claim 1 or 2, wherein the first antibody is a monoclonal antibody and the second antibody is a polyclonal antibody.

5. The method of any one of claims 1 to 4, wherein step (iv) of performing a quantitative analysis of the material to which the second antibody is bound comprises:
(iv-1) capturing the target material to which the second antibody is bound by applying an external magnetic force; and
(iv-2) analyzing the captured target material to which the second antibody is bound using a spectrophotometer.

6. The method of claim 5, wherein step (iv-2) of analyzing the captured target material to which the second antibody is bound using a spectrophotometer is to analyze using an inductively coupled plasma mass spectrometry (ICP-MS) or graphite furnace atomic absorption spectrophotometer.

## Patentansprüche

1. Verfahren zur gleichzeitigen Analyse mehrerer Ziele unter Verwendung mehrerer Metall-Nano-Tags, wobei das Verfahren Folgendes umfasst:
(i) Herstellen einer Analyseplattform, an die ein erster Antikörper gebunden ist, der spezifisch an ein Ziel bindet;
(ii) Umsetzen der Analyseplattform, die den ersten Antikörper umfasst, mit einer Probe, die mehrere Ziele umfasst, und dadurch Bilden einer Analyseplattform, an die Zielmaterialien gebunden sind;
(iii) Reagieren eines zweiten Antikörpers, der spezifisch an ein Ziel bindet, mit der Analyseplattform, in der der erste Antikörper und die Ziele gebunden sind; und
(iv) Durchführen einer quantitativen Analyse des Materials, an das der zweite Antikörper gebunden ist,
wobei:
die Analyseplattform, an die der erste Antikörper gebunden ist, mehrere Arten von Antikörpern umfasst;
die Analyseplattform, an die der erste Antikörper gebunden ist, ein Siliciumdioxid-Nanopartikel ist, der einen metallhaltigen Kern und Siliciumdioxid umfasst, das die Oberfläche des Kerns beschichtet;
das Ziel aus mindestens zwei ausgewählt aus der Gruppe bestehend aus Hepatitis B-Virus (HBV), Hepatitis C-Virus (HCV) und humanem Immundefizienzvirus (HIV) besteht; und
die Analyseplattform, an die der erste Antikörper gebunden ist, mindestens zwei Arten von Siliciumdioxid-Nanopartikeln umfasst, die verschiedene Arten von Metallen enthalten, wobei das Siliciumdioxid-Nanopartikel Au umfasst, wenn das zu analysierende Ziel HBV ist, das Siliciumdioxid-Nanopartikel Gd umfasst, wenn das zu analysierende Ziel HIV ist, und das Siliciumdioxid-Nanopartikel Eu umfasst, wenn das zu analysierende Ziel HCV ist.

2. Verfahren nach Anspruch 1, wobei der zweite Antikörper an ein Siliciumdioxid-Nanopartikel gebunden ist, das einen magnetischen metallhaltigen Kern und Siliciumdioxid, das die Oberfläche des Kerns beschichtet, umfasst.

3. Verfahren nach Anspruch 1 oder 2, wobei der erste Antikörper und der zweite Antikörper gleich sind.

4. Verfahren nach Anspruch 1 oder 2, wobei der erste Antikörper ein monoklonaler Antikörper und der zweite Antikörper ein polyklonaler Antikörper ist.

5. Verfahren nach einem der Ansprüche 1 bis 4, wobei Schritt (iv) des Durchführens einer quantitativen Analyse des Materials, an das der zweite Antikörper gebunden ist, Folgendes umfasst:
(iv-1) Einfangen des Zielmaterials, an das der zweite Antikörper gebunden ist, durch Anwenden einer externen Magnetkraft; und
(iv-2) Analysieren des eingefangenen Zielmaterials, an das der zweite Antikörper gebunden ist, unter Verwendung eines Spektrophotometers.

6. Verfahren nach Anspruch 5, wobei Schritt (iv-2) des Analysierens des eingefangenen Zielmaterials, an das der zweite Antikörper gebunden ist, mit Hilfe eines Spektrophotometers in der Analyse unter Verwendung einer induktiv gekoppelten Plasmamassenspektrometrie (ICP-MS) oder eines Graphitofen-Atomabsorptionsspektrophotometers besteht.

## Revendications

1. Procédé pour analyse simultanée d'une pluralité de cibles en utilisant une pluralité de nano-marqueurs métalliques, comprenant :
(i) la préparation d'une plate-forme d'analyse à laquelle un premier anticorps, qui se lie spécifiquement à une cible, est lié ;
(ii) la réaction de la plate-forme d'analyse comprenant le premier anticorps avec un échantillon comprenant une pluralité de cibles et ainsi la formation d'une plate-forme d'analyse à laquelle des matériaux cibles sont liés ;
(iii) la réaction d'un second anticorps, qui se lie spécifiquement à une cible, avec la plate-forme d'analyse dans laquelle le premier anticorps et des cibles sont liés ; et
(iv) la réalisation d'une analyse quantitative du matériau à laquelle le second anticorps est lié,
dans lequel :
la plate-forme d'analyse à laquelle le premier anticorps est lié comprend une pluralité de types d'anticorps ;
la plate-forme d'analyse à laquelle le premier anticorps est lié est une nanoparticule de silice, comprenant un noyau comprenant du métal et de la silice qui enduit la surface du noyau ;
le cible est au moins deux sélectionnés parmi le groupe constitué de : virus d'hépatite B (VHB), virus d'hépatite C (VHC), et virus d'immunodéficience humaine (VIH) ; et
la plate-forme d'analyse à laquelle le premier anticorps est lié comprend au moins deux types de nanoparticules de silice contenant différents types de métaux, dans lequel la nanoparticule de silice comprend Au lorsque la cible destinée à être analysée est VHB, la nanoparticule de silice comprend Gd lorsque la cible destinée à être analysée est VIH, et la nanoparticule de silice comprend Eu lorsque la cible destinée à être analysée est VHC.

2. Procédé selon la revendication 1, dans lequel le second anticorps est lié à une nanoparticule de silice, qui comprend un noyau magnétique comprenant du métal et de la silice qui enduit la surface du noyau.

3. Procédé selon la revendication 1 ou 2, dans lequel le premier anticorps et le second anticorps sont les mêmes.

4. Procédé selon la revendication 1 ou 2, dans lequel le premier anticorps est un anticorps monoclonal et le second anticorps est un anticorps polyclonal.

5. Procédé de l'une quelconque des revendications 1 à 4, dans lequel l'étape (iv) de la réalisation d'une analyse quantitative du matériau à laquelle le second anticorps est lié comprend :
(iv-1) la capture du matériau cible à laquelle le second anticorps est lié en appliquant une force magnétique externe ; et
(iv-2) l'analyse du matériau cible capturé à laquelle le second anticorps est lié en utilisant un spectrophotomètre.

6. Procédé de la revendication 5, dans lequel l'étape (iv-2) de l'analyse du matériau cible capturé à laquelle le second anticorps est lié en utilisant un spectrophotomètre consiste à analyser en utilisant une spectrométrie de masse couplée à plasma inductif (ICP-MS) ou un spectrophotomètre d'absorption atomique à four graphite.
